# FASCICULE DE BREVET EUROPEEN

(11) **EP 1 185 669 B1**
(45) Date de publication et mention de la délivrance du brevet: **18.08.2004**
(21) Numéro de dépôt: 00940461.7
(22) Date de dépôt: 08.06.2000
(51) Int. Cl.: C12N 15/53, C12N 15/82, C12N 9/02, C12N 5/10, C12Q 1/68, A01H 5/00, A01H 5/10

(54) **PROMOTEUR PERMETTANT L'EXPRESSION DE TRANSGENES DANS TOUTE LA PLANTE HORMIS DANS LA GRAINE**
PROMOTOR, WELCHER DIE EXPRESSION VON TRANSGENEN IN DER GANZEN PFLANZEN AUSSER DEM SAMEN ERLAUBT
PROMOTER ENABLING TRANSGENE EXPRESSION IN THE WHOLE PLANT EXCEPT IN THE SEED

(30) Priorité: 10.06.1999 FR 9907362
(43) Date de publication de la demande: 13.03.2002
(73) Titulaire: INSTITUT NATIONAL DE LA RECHERCHE AGRONOMIQUE, 75007 Paris (FR)
(72) Inventeur: DUBREUCQ, Bertrand, F-75015 Paris (FR); LEPINIEC, Loic, F-91440 Bures-sur-Yvette (FR); CABOCHE, Michel, F-78310 Maurepas (FR)
(74) Mandataire: Warcoin, Jacques
(86) Numéro de dépôt international: PCT/FR2000/001574
(87) Numéro de publication internationale: WO 2000/077223

(56) Documents cités:
- WO-A-97/30582
- WO-A-98/44138
- GUIVARC'H ANNE MAURO CARNEIRO ET AL: "Tissue-specific expression of the rolA gene mediates morphological changes in transgenic tobacco." PLANT MOLECULAR BIOLOGY 1996, vol. 30, no. 1, 1996, pages 125-134, XP002132241 ISSN: 0167-4412
- LIN X. ET AL.: "AC 003096" EMBL DATABASE, 14 novembre 1997 (1997-11-14), XP002148976 Heidelberg
- MITCHELL A G ET AL: "Fah1p, a Saccharomyces cerevisiae cytochrome b5 fusion protein, and its Arabidopsis thaliana homolog that lacks the cytochrome b5 domain both function in the alpha-hydroxylation of sphingolipid-associated very long chain fatty acids." JOURNAL OF BIOLOGICAL CHEMISTRY, (1997 NOV 7) 272 (45) 28281-8., XP002132243 cité dans la demande

## Description

La présente invention concerne l'isolement et la caractérisation d'un promoteur qui permet l'expression de transgènes dans la plante adulte, à des fins d'amélioration du développement de la plante, sans que le produit de ce transgène soit présent dans la graine mature et sèche. L'invention a également trait aux plantes transgéniques comportant un gène d'intérêt fusionné à ladite séquence promotrice.

Les techniques de biologie moléculaires permettent actuellement de modifier le patrimoine génétique des végétaux pour en changer les composantes qui contrôlent la production, la qualité ou la santé. La spécificité d'expression des transgènes introduits repose essentiellement sur l'utilisation de séquences promotrices de plantes ou de microorganismes. La recherche de promoteurs spécifiques est donc d'une importance capitale pour les biotechnologies végétales. Les graines constituent une composante importante de l'agriculture, en tant que semences, mais également dans l'alimentation ou l'industrie de transformation. A ce titre, la présence de protéines et produits nouveaux dans la graine peut poser des problèmes. Il apparaît donc intéressant de pouvoir disposer d'un promoteur actif dans tous les tissus végétatifs, mais inopérant dans les graines.

Les caractéristiques d'une graine vont dépendre des interactions entre la maturation, sous contrôle d'un programme génétique spécifique, et des conditions de l'environnement qui conditionnent pour une bonne part la production ultérieure. Les mécanismes régulateurs de ces phénomènes restent cependant largement incompris. Il existe donc un réel intérêt à maintenir une bonne qualité des lots de semences. Or, le développement des plantes transgéniques posent de nouveaux problèmes, notamment liés à l'expression de gènes hétérologues dans les graines desdites plantes. En effet, la présence de protéines ou de polypeptides dans les graines peut avoir des conséquences néfastes sur leur capacité à germer ou sur leur qualité. En outre, si la population se familiarise de plus en plus avec l'idée que les plantes comestibles peuvent être modifiées génétiquement, des graines comestibles contenant le produit de transgènes pourraient ne pas être facilement acceptées.

Ainsi, l'objectif à la base de la présente invention est d'identifier un promoteur qui permettrait une expression forte d'un transgène dans tous les tissus des plantes sauf dans la graine.

A cet effet, le piégeage de promoteur (« promoter trapping »), un outil puissant pour disséquer des processus développementaux (Topping and Lindsey 1995 pour revue), a été mise en oeuvre. Cette stratégie est basée sur l'utilisation d'un vecteur de transformation des plantes possédant, à l'une de ses extrémités, un gène rapporteur (GUS ou GFP le plus souvent) sans promoteur. Si l'insertion se réalise dans une région codante et si la séquence du gène rapporteur est en phase, il y aura fusion traductionnelle entre la protéine endogène et la protéine du gène marqueur. Les stratégies de piégeage de gène présentent un avantage majeur vis-à-vis de la mutagenèse insertionnelle classique car le phénotype (expression du gène rapporteur GUS) est dominant. Cette dominance du phénotype (GUS) permet de suivre les allèles mutés à l'état hétérozygote. Ceci est très intéressant pour l'étude de mutations qui sont létales à l'état homozygote. Cette approche permet également de caractériser un gène par son expression.

Il a été trouvé lors de accomplissement de la présente invention, qu'une insertion d'un gène rapporteur dans le gène codant pour une protéine de type « fatty acid hydroxylase » (FAH) d'Arabidopsis conduit à une expression dans tous les tissus de la plante sauf dans la graine. Ce type de promoteur est d'un très grand intérêt pour des applications biotechnologiques. Il permet de faire exprimer une protéine d'intérêt dès l'imbibition dans tous les tissus de la plante avec un niveau d'expression élevé, hormis dans la graine. On peut donc, par exemple, protéger une plante contre de nombreux stress biotiques ou abiotiques sans modifier le contenu de sa graine. On peut également faire exprimer une séquence antisens dirigée contre un gène cible dans tous les tissus sauf dans la graine.

### Description

Ainsi, la présente invention concerne une séquence promotrice permettant l'expression d'un gène d'intérêt dans les tissus d'une plante hormis dans la graine en maturation et dans la graine sèche. ladite séquence comprenant une séquence ayant au moins 80% d'identité avec la séquence ou une portion de la séquence du promoteur du gène de la FAH d'Arabidopsis.

De préférence, cette séquence comprend une séquence ayant au moins 80% d'identité avec la séquence ou une portion de la séquence SEQ ID N° 1.
On entend par « % d'identité » le pourcentage de nucléotides identiques qui peut facilement être calculé par l'homme du métier en utilisant un programme informatique de comparaison de séquence tel que le programme DNASIS (Version 2.5 pour Windows ; Hitachi Software Engineering Co., Ltd, South San Francisco, CA) en utilisant les paramètres standards décrits dans le manuel du fabriquant, incorporé dans la description par référence.
Dans ce contexte, les séquences et les pourcentages d'identité peuvent également être obtenus en utilisant les ressources informatiques internet. On peut citer le programme Blast (WWW.ncbi.nlm.nih.gov) et le programme FastDB avec les paramètres suivants « Mismatch penalty 1.00 ; Gap Penalty 1.00 ; Gap Size Penalty 0.33 ; joining penalty 30.0. Ces algorithmes sont présentés dans Current Methods in Sequencing and synthesis Methods and Applications, pages 127-149, 1988, Ala R. Liss, Inc, incorporé dans la description par référence
On peut également définir les séquences ayant 80% d'identité comme étant des séquences s'hybridant à la séquence SEQ ID N°1 avec des conditions de stringence fortes. Ces conditions sont présentées dans Sambrook et al. Molecular Cloning A Laboratory Manual (Cold Spring Harbor Press, 1989) aux paragraphes 11.1 à 11.61, incorporés dans la description par référence.

Avantageusement, la séquence selon l'invention possède la séquence ou une portion de la séquence SEQ ID N°1 suivante :

L'invention a également trait à l'utilisation de portion de la séquence SEQ ID N° 1 pour l'identification de fragments capables de promouvoir l'expression d'un gène d'intérêt dans une plante hormis dans la graine. Il est ainsi possible de définir la région minimale de la séquence du promoteur du gène de la FAH pour assurer une expression efficace. En ce sens, le promoteur peut être modifié par addition de séquences telles des enhancers, par délétion de régions non essentielles et/ou non désirées. Le promoteur peut comprendre des séquences synthétiques et/ou naturelles.

L'invention concerne un procédé permettant l'isolement et la caractérisation du promoteur du gène de la FAH chez les plantes comprenant les étapes suivantes :
a) Utilisation d'une amorce comprenant une séquence ayant au moins 80% d'identité avec une séquence possédant au moins 10 nucléotides consécutifs de la séquence SEQ ID N°5 ou une séquence complémentaire, une amorce qui s'hybride dans des conditions stringentes fortes à toute séquence codante pour la SEQ ID N°4 ou une séquence avant au moins 80% d'identité avec une séquence possédant au moins 10 nucléotides consécutifs de la séquence génomique du gène de la FAH d'Arabidopsis accessible sous le numéro AC003096 ou une séquence complémentaire, pour l'isolement et/ou l'amplification de la séquence en amont de l'extrémité 5' du gène de la FAH,
b) Clonage et séquençage de la séquence obtenue à l'étape a).

La SEQ ID N°5 correspond à la séquence codante du gène de la FAH d'Arabidopsis :
DEFINITION : ADNc complet de « Arabidopsis thaliana fatty acid hydroxylase Fahlp » (FAH1)
ACCESSION: AF021804
ORGANISME : Arabidopsis thaliana, Eukaryota; Viridiplantae; Streptophyta; Embryophyta; Tracheophyta ; Euphyllophytes; Spermatophyta; Magnoliophyta; Eudicotyledons; Rosidae; Brassicales; Brassicaceae.
Référence : Mitchell,A.G. and Martin,C.E, (1997). Fahlp, a saccharomyces cerevisiae cytochrome b5 fusion protein, and its arabidopsis thaliana homolog that lacks the cytochrome b5 domain both function in the alpha-hydroxylation of sphingolipid-associated very long chain fatty acids ; J. Biol. Chem. 272 (45), 28281-28288 MEDLINE 98019193

Il est également possible d'utiliser une amorce comprenant une séquence ayant au moins 80% d'identité avec une séquence possédant au moins 10 nucléotides consécutifs de la séquence génomique du gène de la FAH d'Arabidopsis (introns et exons) qui est accessible à l'homme du métier sous le numéro AC003096 ou une amorce qui s'hybride dans des conditions stringentes fortes à toute séquence codante pour la SEQ ID N°4 (Arabidopsis thaliana, fatty acid hydroxylase Fahlp) suivante :

Ainsi, la séquence promotrice permettant l'expression d'un gène d'intérêt dans les tissus d'une plante, hormis dans la graine en maturation et dans la graine sèche, peut également être caractérisée en ce qu'elle comprend une séquence ayant au moins 80% d'identité avec la séquence ou une portion de la séquence du promoteur du gène de la FAH susceptible d'être obtenue à partir du procédé décrit précédemment.

Un autre aspect de l'invention porte sur une cassette d'expression qui comporte une séquence d'intérêt fusionnée à une séquence comprenant une séquence promotrice telle que définie ci-dessus. Ladite séquence d'intérêt peut coder pour un ARN, une protéine ou un polypeptide qui protège la plante contre un stress biotique ou abiotique.
La cassette peut permettre la co-suppression de l'expression d'un gène caractérisée en ce que ladite séquence d'intérêt code pour une protéine ou un polypeptide capable de se substituer à la fonction d'une protéine ou d'un polypeptide endogène. La séquence d'intérêt peut également coder pour une séquence antisens dirigée contre un gène cible. Ceci permet en couplant avec la surexpression ectopique d'un gène d'intérêt dans les graines d'empêcher toute expression de ce gène dans d'autres tissus. l'antisens n'y étant pas exprimé. Ceci s'avère tout à fait utile dans le cas où l'on souhaite surexprimer une protéine dans les graines sans perturber le développement d'autres tissus de la plante.
La cassette selon l'invention peut comprendre en outre un gène marqueur de sélection, une séquence leader contrôlant le transit, la sécrétion ou le ciblage du produit d'expression dans différents organelles une séquence signal de la terminaison de la transcription et de la traduction.

On entend par « gène d'intérêt» ou « transgène» dans le cadre de l'invention, un gène notamment sélectionné parmi les gènes codant pour une protéine ou un polypeptide qui protège la plante contre un stress biotique ou abiotique, les gènes perturbateurs codant pour un produit capable de se substituer et/ou d'inhiber la fonction ou l'expression d'un ARNm, d'une protéine ou d'un polypeptide endogène. On peut citer par exemple les gènes codant pour des ribozymes contre des ARNm endogènes, des gènes dont le produit de la transcription est au moins complémentaire en partie à un ARNm endogène cible (EP 240 208 incorporé dans la description par référence). On peut également citer des gènes, dont le produit de la transcription est identique ou similaire aux transcripts de gènes endogènes, qui sont capables d'inhiber par co-suppression l'expression desdits gènes endogènes (Napoli C. et al., 1990, The Plant Cell, 2, 279-289, cité dans la description par référence). Bien entendu, le gène selon l'invention peut coder pour une enzyme impliquée dans le métabolisme de sorte à faire produire ou à favoriser la biosynthèse de métabolites, notamment de métabolites utiles pour l'alimentation humaine ou animale ou pouvant affecter le développement. La séquence promotrice selon l'invention peut induire l'expression d'un gène étranger et être utilisée dans différent types de plantes. Le terme « gène étranger » ou « transgène » est également compris comme définissant toute région d'ADN codante ou non (protéine, polypeptide, antisens, ARN catalytique, viroïde etc...). Une protéine d'intérêt pour le développement et la production de la plante peut être produite de façon constitutive dans tous les organes de la plante en utilisant ce promoteur sans que la composition de la graine soit affectée. Les protéines d'intérêt sont. de manière non exhaustive. celles qui permettent une meilleure protection de la plante vis-à-vis
- des stress biotiques : protection contre les pathogènes, bactéries, champignons, insectes, nématodes, parasites ou ravageurs, protection contres les virus et pathogènes intacellulaires, en particulier ceux qui ne sont pas transmis par les graines
- des stress abiotiques : protection contres la chaleur et le froid, le gel, les stress hydriques tels que la sécheresse ou à l'inverse l'anoxie, la pollution (ozone, SO2), la photoinhibition et les stress lumineux, la verse, la phytorémédiation, ou les stress nutritionnels occasionnés par une carence ou excès d'un élément nutritif (en particulier un stress salin).
Tout gène d'intérêt peut donc être placé sous le contrôle de la séquence promotrice isolée. Pour l'expression dans les plantes, ce gène peut comporter également des séquences 3' non transcrites possédant des signaux de polyadénylation actifs chez les plantes. Ces séquences peuvent, par exemple, être celles codant la partie 3'transcrite non traduite du gène l'ARM' 35S du virus de la mosaïque de choux fleur (35S CaMV) ou la région 3' non-traduite du gène codant la synthèse de la nopaline (NOS) du plasmide Ti d'Agrobacterium tumefaciens.

Le gène d'intérêt selon l'invention peut également être un gène contrôlant le développement tel que par exemple un gène impliqué dans le métabolisme des hormones, dans la transduction des signaux, ou dans le contrôle du cycle cellulaire.

Un autre aspect de l'invention a trait à un vecteur, notamment un vecteur plasmidique, comprenant une cassette d'expression telle que définie ci-dessus.
L'invention a également pour objet une cellule de plante transformée avec la cassette ou avec un vecteur comprenant ladite cassette et un kit de transformation de plante comprenant ladite cassette ou ledit vecteur.

La préparation des plasmides, la construction des gènes chimériques et des cassettes d'expression, les restrictions de l'ADN par des endonucléases, la transformation et la confirmation des transformations sont réalisés selon les protocoles standards (Sambrook et al. 1989. Molecular Cloning Manual Cold Spring Harbor Laboratory. incorporés dans la description par référence).
La construction des vecteurs utilisables pour les expériences de transformation fait partie des techniques de biologie moléculaire connues et pratiquées de façon routinière dans ce champ d'applications.

Un aspect supplémentaire de l'invention porte sur un procédé de préparation de plantes transgéniques dans lesquelles un gène d'intérêt est exprimé dans tous les tissus sauf dans la graine en maturation et dans la graine sèche caractérisé en ce qu'il comprend les étapes suivantes :
a) Transfert d'une cassette ou d'un vecteur selon l'invention dans des cellules de plantes,
b) Mise en culture des cellules transformées obtenues à l'étape a) de manière à obtenir lesdites plantes transgéniques.

Le transfert de l'ADN à l'intérieur des cellules de plante, notamment des cellules de l'albumen ou des cellules totipotentes dérivées d'embryons immatures, peut être effectué par les techniques standards (Plant Cell Report, 10, 595, 1992), en particulier par transfert via Agrobacterium (Plant J., 1994. 6, 271), par électroporation (Nature, 1987,327, 70), laserporation (Barlev Genetics. 1991 VI, 231), par polyéthylène glycol, ou par la méthode biolistique dite « gun particule » (Nature, 1987, 327, 70). De manière générale, pour les vecteurs de transformations via une agrobactérie (infiltration in planta Bechtold et al. 1993), les vecteurs de transformation portent des marqueurs de sélection, des bordures d'ADN-T, des sites de clonage, des fonctions de réplication, ainsi que d' autres éléments si nécessaire au bon transfert des transgènes (Bouchez et al. 1993). Les publications ci-dessus mentionnée sont incorporées dans la description par références.

La présente invention a élément pour objet une plante transgénique susceptible d'être obtenue en mettant en oeuvre le procédé mentionnée ci-dessus.

Par « plante susceptible d'être obtenue », on entend toute plante exprimant un transgène dans ses tissus sauf dans les graines matures et sèches, ladite plante possédant un promoteur selon l'invention. Les plantes obtenues par tout procédé équivalent conduisant au même résultat sont également objet de l'invention. La liste des plantes chez lesquelles cette séquence promotrice peut être utilisée inclue plus particulièrement les plantes qui sont utiles pour toute industrie. On peut citer par exemple le colza, les crucifères, le maïs, le soja, le blé, le tournesol, le pois, les plantes ornementales, et les arbres.

Ainsi, l'invention concerne une plante, telle que définie ci-dessus, qui exprime dans ses tissus, sauf dans les graines, un gène dont le produit (ARN ou protéine) protège la plante contre un stress biotique ou abiotique, une séquence antisens dirigée contre un gène cible, une protéine ou un polypeptide capable de se substituer à la fonction d'une protéine ou d'un polypeptide endogène ou une séquence codante pour une protéine impliquée dans la biosynthèse de métabolites ou un gène contrôlant le développement tel que par exemple un gène impliqué dans le métabolisme des hormones, dans la transduction des signaux, ou dans le contrôle du cycle cellulaire. La plante selon l'invention peut également exprimer une protéine d'intérêt sous le contrôle d'un promoteur autre que le promoteur du gène de la FAH et une séquence antisens capable d'inhiber l'expression de ladite protéine d'intérêt sous le contrôle du promoteur du gène de la FAH, de telle sorte que le gène d'intérêt n'est exprimé que dans les graines.

Les graines obtenues à partir d'une plante transgénique selon l'invention, qui ne contiennent donc pas le produit d'expression du transgène, sont visées par la présente invention, ainsi que leur utilisation en toute industrie.

Pour la suite de la description, on se référera aux légendes des figures présentées ci-dessous.

### Légendes

**Figure 1 : Structure intro /exon de l'ARNm du gène de la FAH**
   Les rectangles avec les rayures représentent les introns.
   L'échelle est présentée sur la figure.
   T29F13 est un bac et TA1234un ADNc.
**Figure 2 Structure de la région du géne FAH**
   PFAH upper et Al représentent les amorces utilisées pour séquencer le promoteur.
   Les rectangles avec les rayures représentent la partie 5' transcrite non traduite.
   L'échelle est présentée sur la figure.
**Figure 3 : Carte du plasmide pB1 101**
   Carte du plasmide pB1 101 contenant le promoteur pFAH utilisé.

### Exemple 1 : Clonage du promoteur.

### Matériels et méthodes

### Isolement de la région promotrice de la FAH.

La méthode utilisée pour l'extraction d' ADN génomique d' Arabidopsis s'inspire de celle décrite par Doyle et Doyle (1990). Le principe repose sur les propriétés détergentes du bromure de cétyltriméthylammonium (CTAB; Sigma Chemical Co., USA) permettant la dénaturation spécifique des macromolécules protéiques et polysaccharidiques. Environ 2g de matériel végétal (plantules cultivées in vitro, âgées de 1 à 2 semaines) sont finement broyés dans l'azote liquide et transférés dans un tube de 50ml de type FALCON (Costar, USA), contenant 7.5ml de tampon d'extraction préchauffé à 65 °C. L'extraction s'effectue à 65°C pendant 30 minutes, sous agitation régulière. Les protéines dénaturées par le β-mercaptoéthanol et le CTAB du tampon sont ensuite extraites dans un volume de chloroforme puis éliminées après centrifugation (4430g. 10min). Les acides nucléiques du surnageant sont précipités par un volume d'isopropanol en présence d'acétate de sodium 3M (1/10, v/v), centrifugés (7900g, 10min) puis rincés à l'éthanol 70%. Le culot est repris en tube Eppendorf dans 100µl d'eau et les acides ribonucléiques sont éliminés par l'adjonction de 3µl de Rnase A à 10mg/ml (Sigma Chemical Co.. USA). L'ADN est déprotéinisé puis à nouveau précipité à l'éthanol absolu. Après centrifugation en tube Eppendorf, le culot est lavé. séché, repris dans 50 à 100µl d'eau et conservé à -20°C avant analyses.

### Amplification de l'ADN génomique.

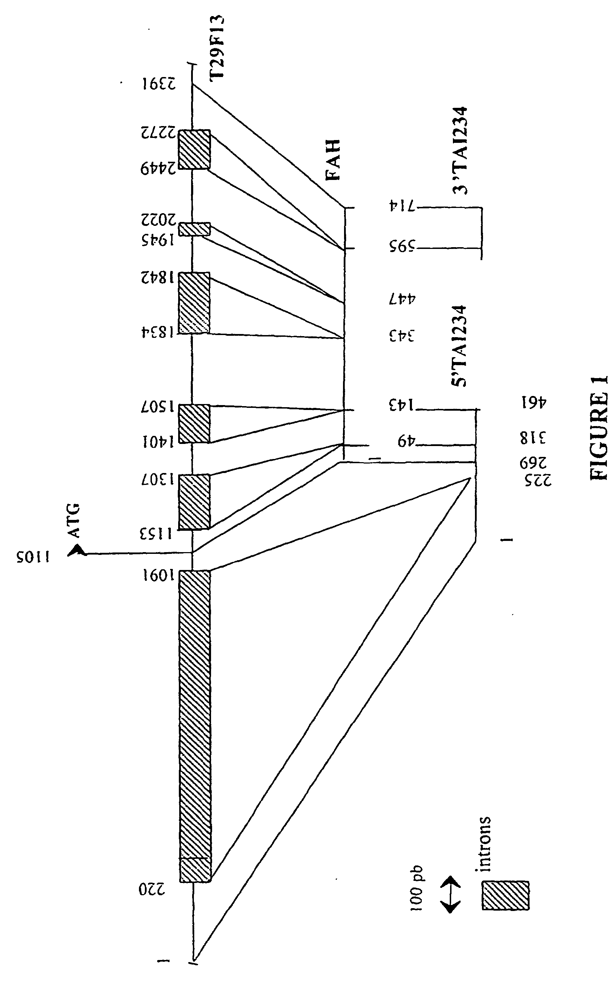

La séquence promotrice a été amplifiée en utilisant la technologie de la PCR qui est une technique connue (Sambrook et al. 1989) . Des amorces correspondantes à la partie 5'(upper) et 3'(lower) de la séquence promotrice ont été dérivées de la séquence génomique du BAC T29F13 (AC003096) (Voir figure 1). De l'ADN génomique d'une lignée de type sauvage (Ler) a été utilisé comme matrice pour l'amplification de la partie promotrice. Les réactions d'amplification ont été menées sur un thermocycleur (MJ Research PTC100 -96), dans des tubes de 0,2ml (Prolabo) contenant le mélange suivant:
1 µl (10ng) ADN, 2µl Tampon 10 x (BRL), 2µl MgCl2 25mM, 0,8µl dNTP 5mM, 1µl Amorce 1 (10pmole/µl), 1µl Amorce 2 (10pmole/µl), 0,5µl (1U) Taq DNA polymérase (5U/µl), H2O qsp 20µl

### Transformation bactérienne.

Les génotypes de bactéries utilisés pour la réalisation des expériences sont :

E. Coli souche DH12S (φ80 dlacZ ΔM15 mcrA Δ(mrr-hsdRMS-mcrBC) araD139 Δ(ara,leu)7697 ΔlacX74 galU galK rpsL deoR nupG recAl/F'proAB+ laclq ZΔM15).

### Agrobactèrium tumefaciens pmp90C58CE

La transformation des bactéries (E.coli souche DH12S) par un plasmide recombiné est réalisée par électroporation (Potter, 1993). Dans une cuve à électroporation (1ml, largeur 0.1cm), 2µl de la réaction de ligature sont mélangés avec 50µl de bactéries décongelées et maintenues dans la glace. La cuve est ensuite placée dans un électroporateur (Gene Pulser II System: BIO-RAD, FRANCE) et une tension de 1.25kV est appliquée pendant un temps qui est fonction de la résistance (200Ω) et de la capacité (25µF) du circuit. Un ml de milieu SOC est ajouté pour favoriser la croissance des bactéries et le tout est incubé dans un tube de 10ml pendant 2 heures à 37°C. sous agitation rotative (220rpm). Les bactéries transformées sont ensuite étalées sur des boîtes contenant du milieu LB solide supplémenté avec l'antibiotique adéquat et incubées à 37°C pendant une nuit. La sélection des bactéries transformées par le plasmide pMeca recombiné se fait par 0,04mg/ml d'ampicilline en présence de 0.2mg/ml de X-Gal et de 0.05mg/ml d'IPTG. Pour les autres plasmides recombinés, la sélection des bactéries s'effectue sur un milieu LB avec l'antibiotique adéquat à une concentration finale de 0,04mg/ml.

### Activité β-glucuronidase.

Pour les graines, un semis est effectué sur deux épaisseurs de papier Whatman 1M de 4,7cm (Maindstone, Angleterre) imbibé par 2ml d'eau stérile. Après 48h d'imbibition dans une boîte saturée en eau, les graines sont raclées et placées dans un tube Eppendorf auquel sont ajoutés 100µl de tampon d'infiltration (100mM de Tampon phosphate pH7,2, 10mM EDTA, Triton X100 0,1% v/v) supplémenté en X-Gluc (5-Bromo-4-Chloro-3-indolyl-β-D-Glucuronic Acid). Le X-Gluc est dissout dans du DMF (diméthylformamide) à une concentration stock: 100µ (10mg/100µl). Le tampon d'infiltration est supplémenté au 1/100e extemporanément avec le stock de X-Gluc. Pour les autres tissus, les échantillons sont placés directement dans le tampon d'infiltration et la coloration est ensuite réalisée selon le même protocole.
L'infiltration est effectuée sous-vide (dans une cloche à vide):
- le vide est cassé 2 fois.
- le vide est maintenu pendant 1 heure, puis les échantillons sont placés à 37°C pour la nuit.

### Résultats

Des analyses préliminaires ont indiqué qu'une enzyme impliquée dans le métabolisme des lipides (la « Fatty Acid Hydroxylase » : FAH) pourrait avoir une expression correspondant au type de promoteur ayant les caractéristiques recherchées.

La séquence du gène en question a été obtenue grâce aux séquences provenant du séquençage systématique du génome d'Arabidopsis thaliana et se trouve être localisée sur le BACT29F13. Une séquence exprimée (EST TAI234) a été identifiée dans les bases de données et semble correspondre à une séquence pleine longueur de l'ARNm de la FAH. Ceci a permis l'identification de la séquence 5' transcrite non traduite et de l'emplacement prévu de la séquence promotrice. La structure intron/exon a été déduite, au niveau de la partie transcrite non traduite, de l'alignement du BAC avec l'EST TAI234 (figure 1).

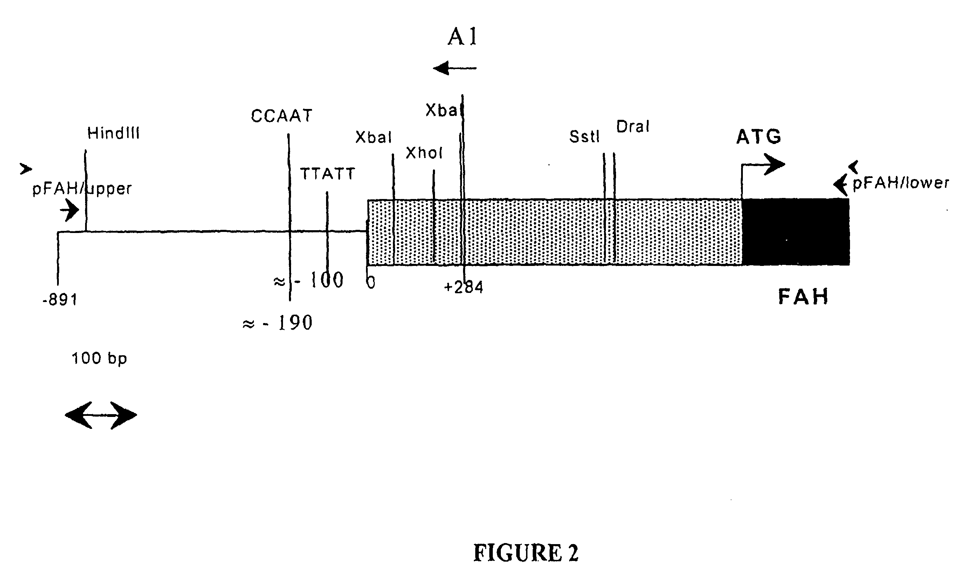

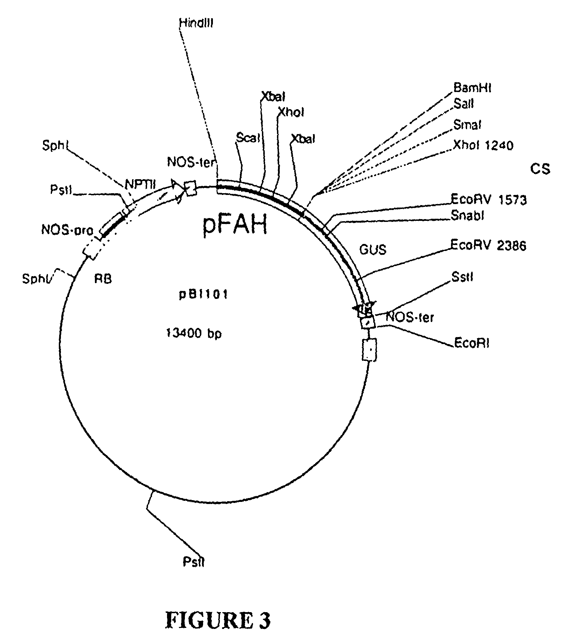

Le promoteur a été amplifié par PCR à partir de l'amorce pFAH/upper et l'amorce Al, placé dans la partie 5' transcrite non traduite (figure 2). Une étude de la séquence a montré que la séquence amplifiée possède une boîte TATA putative à -100pb du site d'initiation présumé de la transcription (d'après l'ADNc pleine longueur) et une boîte CCAAT à -190pb de ce même début de transcription. Le fragment de PCR amplifié (932pb) a été cloné dans un vecteur pGEM-T (PROMEGA), séquence, puis introduit dans un vecteur binaire (pBI101, Clontech) contenant un gène rapporteur GUS sans promoteur (figure 3). Cette construction a ensuite été introduite par transformation in planta, via Agrobacterium, dans des plantes de type sauvage (écotype Ws). Treize transformants primaires ont été obtenus, qui ont été testés pour leur activité GUS pendant leur développement.

### Exemple 2 : Expression du gène rapporteur sous le contrôle du promoteur de gène de la FAH.

L'expression est forte dès 20 heures après le début de l'imbibition dans l'embryon. Durant le développement, l'expression est forte dans tous les tissus, avec une certaine préférence pour les tissus vasculaires.
Ces résultats démontrent que la séquence promotrice isolée confère bien un profil d'expression très spécifique au gène rapporteur utilisé (GUS), le promoteur est actif tout au long du développement de la plante, dans tous les tissus testés (feuilles, fleurs. tiges, racines etc...) sauf dans la graine en cours de maturation (Voir tableau I ci-dessous).

**Tableau 1 :**

| Profil d'expression du gène rapporteur GUS | | | | | | | |
|---|---|---|---|---|---|---|---|
| | cotylédons | feuille adulte | Racines | fleur | silique | graines en germination | Graines sèches |
| 1 | ++ | +++ | ++ | ++ | +++ | +++ | - |
| 2 | +++ | +++ | ++ | ++ | +++ | +++ | - |
| 3 | +++ | +++ | ++ | ++ | nd | ++ | - |
| 4 | +++ | +++ | ++ | ++ | nd | ++ | - |
| 5 | +++ | +++ | ++ | ++ | nd | +++ | - |
| 6 | + | +++ | ++ | ++ | nd | +++ | - |
| 7 | +++ | +++ | ++ | ++ | +++ | + | - |
| 8 | ++ | +++ | ++ | ++ | +++ | +++ | - |
| 9 | +++ | +++ | ++ | ++ | +++ | ++ | - |

L'expression du marqueur confirme la fonctionnalité du promoteur et sa spécificité. Ce type de promoteur est donc d'un très grand intérêt pour des applications biotechnologiques, telles que l'expression d'une toxine anti-insecte (type Bt) dans les plantes et l'expression de tout transgène permettant d'améliorer, de manière quantitative ou qualitative, le développement et la croissance de la plante sans que la protéine codée par le transgène soit présente dans la graine.

### REFERENCES

Bechlold N., Ellis J. and Pelletier G. (1993). In planta Agrobacterium mediated gene transfer by infiltration of adult Arabidopsis thaliana plants. C.R. Acad. Sci. Paris, Sciences de la vie; 316: 1194-9
Bouchez D., Camilleri C. Caboche M. (1993). A binary vector based on Basta résistance for in planta transformation of Arabidopsis thaliana. C.R. Acad. Sci. Paris, Sciences de la vie: 316: 1188-1193
Doyle J.J. and Doyle .1. L. (1990). Isolation of plant DNA from fresh tissue. Focus; 12: 13-15
Sambrook J.. Fritsch E. F. and Maniatis T. (1989).; Cold Spring Harbor Laboratory, Cold Spring Harbor N.Y.

## Revendications

1. Séquence promotrice permettant l'expression d'un gène d'intérêt dans les tissus d'une plante, hormis dans la graine en maturation et dans la graine sèche, **caractérisée en ce qu'**elle comprend une séquence ayant au moins 80% d'identité avec la séquence SEQ ID N°1 du promoteur du gène de la FAH d'Arabidopsis.

2. Séquence selon la revendication 1 **caractérisée en ce qu'**elle comprend une séquence s'hybridant avec des conditions de fortes stringence à la séquence SEQ ID N°1.

3. Séquence selon la revendication 1 **caractérisée en ce qu'**elle comprend la séquence SEQ ID N°1.

4. Procédé permettant l'isolement et la caractérisation du promoteur du gène de la FAH chez les plantes comprenant les étapes suivantes :
a) Utilisation d'une amorce comprenant une séquence ayant au moins 80% d'identité avec une séquence possédant au moins 10 nucléotides consécutifs de la séquence SEQ ID N°5 ou une séquence complémentaire, une amorce qui s'hybride dans des conditions stringentes fortes à toute séquence codante pour la SEQ ID N°4 ou une séquence ayant au moins 80% d'identité avec une séquence possédant au moins 10 nucléotides consécutifs de la séquence génomique du gène de la FAH d'Arabidopsis accessible sous le numéro AC003096 ou une séquence complémentaire, pour l'isolement et/ou l'amplification de la séquence en amont de l'extrémité 5' du gène de la FAH,
b) Clonage et séquençage de la séquence obtenue à l'étape a).

5. Utilisation d'une séquence selon l'une des revendications 1 à 3 pour l'identification de fragments de la séquence SEQ ID N°1 permettant l'expression d'un gène d'intérêt dans les tissus d'une plante, hormis dans la graine en maturation et dans la graine sèche.

6. Cassette d'expression **caractérisée en ce qu'**elle comporte une séquence d'intérêt fusionnée à une séquence comprenant une séquence promotrice selon l'une des revendications 1 à 3.

7. Cassette d'expression selon la revendication 6 **caractérisée en ce que** ladite séquence d'intérêt code pour un ARN, une protéine ou un polypeptide qui protège la plante contre un stress biotique ou abiotique, ou qui est impliquée dans le développement notamment dans le métabolisme des hormones, dans la transduction des signaux, ou dans le contrôle du cycle cellulaire.

8. Cassette d'expression selon la revendication 6 permettant la co-suppression d'un gène **caractérisée en ce que** ladite séquence d'intérêt code pour une protéine ou un polypeptide capable de se substituer à la fonction d'une protéine ou d'un polypeptide endogène.

9. Cassette d'expression selon la revendication 6 **caractérisée en ce que** ladite séquence d'intérêt code pour une séquence antisens dirigée contre un gène cible.

10. Cassette d'expression selon la revendication 6 **caractérisée en ce que** ladite séquence d'intérêt code pour une enzyme impliquée dans la production de métabolites par une plante.

11. Vecteur comprenant une cassette d'expression selon l'une des revendications 6 à 9.

12. Cellule de plante transformée avec une cassette selon l'une des revendications 6 à 9 ou un vecteur selon la revendication 11.

13. Kit de transformation de plante comprenant une cassette selon l'une des revendications 6 à 9 ou un vecteur selon la revendication 11.

14. Procédé de préparation de plantes transgéniques dans lesquelles un gène d'intérêt est exprimé dans tous les tissus sauf dans la graine en maturation et dans la graine sèche **caractérisé en ce qu'**il comprend les étapes suivantes :
a) Transfert d'une cassette selon l'une des revendications 6 à 9 ou d'un vecteur selon la revendication 11 dans des cellules de plantes,
b) Mise en culture des cellules transformées obtenues à l'étape a) de manière à obtenir lesdites plantes transgéniques.

15. Procédé selon la revendication 14 **caractérisé en ce que** les cellules sont choisies parmi les cellules embryonnaires provenant d'un embryon immature.

16. Procédé selon l'une des revendications 14 et 15 **caractérisé en ce que** le transfert est effectué en utilisant Agrobacterium, de préférence Agrobacterium tumefaciens.

17. Plante transgénique **caractérisée en ce qu'**elle comprend une cassette selon les revendications 6 à 10.

18. Plante selon la revendication 17 **caractérisée en ce qu'**elle exprime dans ses tissus, sauf dans les graines matures et sèches, un ARN, une séquence antisens dirigée contre un gène cible.

19. Plante selon la revendication 17 **caractérisée en ce qu'**elle exprime dans ses tissus, sauf dans les graines matures et sèches, un ARN, une protéine ou un polypeptide capable de se substituer à la fonction d'une protéine ou d'un polypeptide endogène.

20. Plante selon la revendication 17 **caractérisée en ce qu'**elle exprime une protéine d'intérêt sous le contrôle d'un promoteur autre que le promoteur du gène de la FAH et une séquence antisens capable d'inhiber l'expression de ladite protéine d'intérêt sous le contrôle du promoteur du gène de la FAH, de telle sorte que la protéine d'intérêt n'est exprimée que dans les graines.

21. Plante selon la revendication 17 **caractérisée en ce qu'**elle exprime dans ses tissus. sauf dans les graines matures et sèches, une séquence codante pour une protéine impliquée dans la biosynthèse de métabolites, pour une protéine ou un polypeptide qui protège la plante contre un stress biotique ou abiotique, une protéine contrôlant le développement notamment dans le métabolisme des hormones, dans la transduction des signaux, ou dans le contrôle du cycle cellulaire.

22. Plante selon l'une des revendications 17 à 21 **caractérisée en ce qu'**elle est choisie notamment parmi le colza, les crucifères, le maïs, le soja, le blé, le tournesol, le pois, les plantes ornementales, et les arbres.

23. Graines obtenues à partir d'une plante transgénique selon l'une des revendications 17 à 22 **caractérisées en ce qu'**elles ne contiennent pas le produit d'expression du transgène, lesdites graines comprenant une cassette selon l 'une des revendications 6 à 10 ou un vecteur selon la revendication 11.

## Patentansprüche

1. Promotorsequenz, welche die Expression eines Gens von Interesse in den Geweben einer Pflanze außer in dem in Reifung befindlichen Samenkorn und in dem trockenen Samenkorn erlaubt, **dadurch gekennzeichnet, dass** sie eine Sequenz umfasst, welche wenigstens 80% Identität mit der Sequenz SEQ ID No. 1 des Promotors des Gens der FAH von Arabidopsis aufweist.

2. Sequenz nach Anspruch 1, **dadurch gekennzeichnet, dass** sie eine Sequenz umfasst, die unter Bedingungen hoher Stringenz mit der Sequenz SEQ ID No. 1 hybridisiert.

3. Sequenz nach Anspruch 1, **dadurch gekennzeichnet, dass** sie die Sequenz SEQ ID No. 1 umfasst.

4. Verfahren, welches die Isolierung und Charakterisierung des Promotors des Gens der FAH in Pflanzen, ermöglicht, welches die folgenden Schritte umfasst:
a) Verwendung eines Primers, welcher eine Sequenz umfasst, welche wenigstens 80% Identität mit einer Sequenz, welche wenigstens 10 aufeinanderfolgende Nukleotide der Sequenz SEQ ID No. 5 oder einer komplementären Sequenz aufweist, aufweist, eines Primers, der unter Bedingungen hoher Stringenz mit einer jeglichen SEQ ID No. 4 kodierenden Sequenz oder einer Sequenz, welche wenigstens 80% Identität mit einer Sequenz, die wenigstens 10 aufeinanderfolgende Nukleotide der genomischen Sequenz des Gens der FAH von Arabidopsis, welche unter der Nummer AC003096 zugänglich ist, oder einer komplementären Sequenz aufweist, aufweist, hybridisiert, für die Isolierung und/oder Amplifizierung der Sequenz stromaufwärts von dem 5'-Ende des Gens der FAH,
b) Klonierung und Sequenzierung der in Schritt a) erhaltenen Sequenz.

5. Verwendung einer Sequenz nach einem der Ansprüche 1 bis 3 für die Identifizierung von Fragmenten der Sequenz SEQ ID No.1, welche die Expression eines Gens von Interesse in den Geweben einer Pf0lanze außer in dem in Reifung befindlichen Samenkorn und in dem trockenen Samenkorn erlauben.

6. Expressionskassette, **dadurch gekennzeichnet, dass** sie eine Sequenz von Interesse fusioniert mit einer Sequenz, welche eine Promotorsequenz nach einem der Ansprüche 1 bis 3 umfasst, umfasst.

7. Expressionskassette nach Anspruch 6, **dadurch gekennzeichnet, dass** die Sequenz von Interesse eine RNA, ein Protein oder ein Polypeptid, welche bzw. welches die Pflanze gegen einen biotischen oder abiotischen Stress schützt oder welche bzw. welches an der Entwicklung, insbesondere am Metabolismus der Hormone, an der Transduktion von Signalen oder an der Kontrolle des Zellzyklus, beteiligt ist, kodiert.

8. Expressionskassette nach Anspruch 6, welche die Cosuppression eines Gens erlaubt, **dadurch gekennzeichnet, dass** die Sequenz von Interesse ein Protein oder ein Polypeptid kodiert, welches in der Lage ist, die Funktion eines endogenen Proteins oder Polypeptids zu ersetzen.

9. Expressionskassette nach Anspruch 6, **dadurch gekennzeichnet, dass** die Sequenz von Interesse eine Antisinn-Sequenz, welche gegen ein Zielgen gerichtet ist, kodiert.

10. Expressionskassette nach Anspruch 6, **dadurch gekennzeichnet, dass** die Sequenz von Interesse ein Enzym, welches an der Produktion von Metaboliten durch eine Pflanze beteiligt ist, kodiert.

11. Vektor, welcher eine Expressionskassette nach einem der Ansprüche 6 bis 9 umfasst.

12. Mit einer Kassette nach einem der Ansprüche 6 bis 9 oder einem Vektor nach Anspruch 11 transformierte Pflanzenzelle.

13. Kit zur Transformation von Pflanzen, welcher eine Kassette nach einem der Ansprüche 6 bis 9 oder einen Vektor nach Anspruch 11 umfasst.

14. Verfahren zur Erzeugung von transgenen Pflanzen, in welchen ein Gen von Interesse in allen Geweben außer in dem in Reifung befindlichen Samenkorn und in dem trokkenen Samenkorn exprimiert wird, **dadurch gekennzeichnet, dass** es die folgenden Schritte umfasst:
a) Transfer einer Kassette nach einem der Ansprüche 6 bis 9 oder eines Vektors nach Anspruch 11 in Zellen von Pflanzen,
b) Kultivierung der in Schritt a) erhaltenen transformierten Zellen derart, dass die transgenen Pflanzen erhalten werden.

15. Verfahren nach Anspruch 14, **dadurch gekennzeichnet, dass** die Zellen unter den embryonalen Zellen, welche von einem unreifen Embryo stammen, ausgewählt werden.

16. Verfahren nach einem der Ansprüche 14 und 15, **dadurch gekennzeichnet, dass** der Transfer unter Verwendung von Agrobacterium, vorzugsweise Agrobacterium tumefaciens ausgeführt wird.

17. Transgene Pflanze, **dadurch gekennzeichnet, dass** sie eine Kassette nach den Ansprüchen 6 bis 10 umfasst.

18. Pflanze nach Anspruch 17, **dadurch gekennzeichnet, dass** sie in ihren Geweben außer in den reifen und trockenen Samenkörnern eine RNA, eine Antisinn-Sequenz, welche gegen ein Zielgen gerichtet ist, exprimiert.

19. Pflanze nach Anspruch 17, **dadurch gekennzeichnet, dass** sie in ihren Geweben außer in den reifen und trockenen Samenkörnern eine RNA, ein Protein oder ein Polypeptid, welche in der Lage sind, die Funktion eines endogenen Proteins oder Polypeptids zu ersetzen, exprimiert.

20. Pflanze nach Anspruch 17, **dadurch gekennzeichnet, dass** sie ein Protein von Interesse unter der Kontrolle eines anderen Promotors als des Promotors des Gens der FAH und eine Antisinn-Sequenz, welche in der Lage ist, die Expression des Proteins von Interesse zu inhibieren, unter der Kontrolle des Promotors des Gens der FAH exprimiert derart, dass das Protein von Interesse lediglich in den Samenkörnern exprimiert wird.

21. Pflanze nach Anspruch 17, **dadurch gekennzeichnet, dass** sie in ihren Geweben außer in den reifen und trockenen Samenkörnern eine Sequenz, welche ein Protein, das an der Biosynthese von Metaboliten beteiligt ist, ein Protein oder ein Polypeptid, welches die Pflanze gegen einen biotischen oder abiotischen Stress schützt, ein Protein, welches die Entwicklung, insbesondere im Rahmen des Metabolismus der Hormone, im Rahmen der Transduktion von Signalen oder im Rahmen der Kontrolle des Zellzyklus, kontrolliert, kodiert, exprimiert.

22. Pflanze nach einem der Ansprüche 17 bis 21, **dadurch gekennzeichnet, dass** sie insbesondere unter Raps, den Kreuzblütlern, Mais, Soja, Getreide (Weizen), Sonnenblume, Erbse, den Zierpflanzen und den Bäumen ausgewählt wird.

23. Samenkörner, welche ausgehend von einer transgenen Pflanze nach einem der Ansprüche 17 bis 22 erhalten werden, **dadurch gekennzeichnet, dass** sie das Expressionsprodukt des Transgens nicht enthalten, wobei die Samenkörner eine Kassette nach einem der Ansprüche 6 bis 10 oder einen Vektor nach Anspruch 11 umfassen.

## Claims

1. Promoter sequence which allows the expression of a gene of interest in the tissues of a plant, except in the maturing seed and in the dry seed, **characterized in that** it comprises a sequence having at least 80% identity with the sequence SEQ ID No. 1 of the promoter of the Arabidopsis FAH gene.

2. Sequence according to Claim 1, **characterized in that** it comprises a sequence which hybridizes under high stringency conditions to the sequence SEQ ID No. 1.

3. Sequence according to Claim 1, **characterized in that** it comprises the sequence SEQ ID No. 1.

4. Method for isolating and characterizing the promoter of the FAH gene in plants, comprising the following steps:
a) using a primer comprising a sequence having at least 80% identity with a sequence containing at least 10 consecutive nucleotides of the sequence SEQ ID No. 5 or a complementary sequence, or a primer which hybridizes under high stringency conditions to any coding sequence for SEQ ID No. 4 or a sequence having at least 80% identity with a sequence containing at least 10 consecutive nucleotides of the genomic sequence of the FAH gene of Arabidopsis, accessible under the number AC003096, or a complementary sequence, for isolating and/or amplifying the sequence upstream of the 5' end of the FAH gene,
b) cloning and sequencing of the sequence obtained in step a).

5. Use of a sequence according to one of Claims 1 to 3, for identifying fragments of the sequence SEQ ID No. 1 which allows the expression of a gene of interest in the tissues of a plant, except in the maturing seed and in the dry seed.

6. Expression cassette, **characterized in that** it comprises a sequence of interest fused to a sequence comprising a promoter sequence according to one of Claims 1 to 3.

7. Expression cassette according to Claim 6, **characterized in that** the sequence of interest encodes an RNA, a protein or a polypeptide which protects the plant against a biotic or abiotic stress, or which is involved in development, in particular in hormone metabolism, in signal transduction or in the control of the cell cycle.

8. Expression cassette according to Claim 6, which allows the cosuppression of a gene, **characterized in that** said sequence of interest encodes a protein or polypeptide capable of substituting for the function of an endogenous protein or polypeptide.

9. Expression cassette according to Claim 6, **characterized in that** said sequence of interest encodes an antisense sequence directed against a target gene.

10. Expression cassette according to Claim 6, **characterized in that** said sequence of interest encodes an enzyme involved in the production of metabolites by a plant.

11. Vector comprising an expression cassette according to one of Claims 6 to 9.

12. Plant cell transformed with a cassette according to one of Claims 6 to 9 or a vector according to claim 11.

13. Plant transformation kit comprising a cassette according to one of Claims 6 to 9 or a vector according to Claim 11.

14. Method for preparing transgenic plants in which a gene of interest is expressed in all the tissues except in the maturing seed and in the dry seed, **characterized in that** it comprises the following steps:
a) transferring a cassette according to one of Claims 6 to 9 or a vector according to Claim 11 into plant cells,
b) culturing the transformed cells obtained in step a) so as to obtain said transgenic plants.

15. Method according to Claim 14, **characterized in that** the cells are chosen from embryonic cells originating from an immature embryo.

16. Method according to either of Claims 14 and 15, **characterized in that** the transfer is carried out using Agrobacterium, preferably Agrobacterium.tumefaciens.

17. Transgenic plant **characterized in that** it comprises a cassette according to Claims 6 to 10.

18. Plant according to Claim 17, **characterized in that** it expresses in its tissues, except in the mature and dry seeds, an RNA, an antisense sequence directed against a target gene.

19. Plant according to Claim 17, **characterized in that** it expresses in its tissues, except in the mature and dry seeds, an RNA, a protein or a polypeptide capable of substituting for the function of an endogenous protein or polypeptide.

20. Plant according to Claim 17, **characterized in that** it expresses a protein of interest under the control of a promoter other than the promoter of the FAH gene, and an antisense sequence capable of inhibiting the expression of said protein of interest under the control of the promoter of the FAH gene, such that the protein of interest is expressed only in the seeds.

21. Plant according to Claim 17, **characterized in that** it expresses in its tissues, except in the mature and dry seeds, a coding sequence for a protein involved in the biosynthesis of metabolites, for a protein or a polypeptide which protects the plant against a biotic or abiotic stress, or for a protein which controls development, in particular in hormone metabolism, in signal transduction or in the control of the cell cycle.

22. Plant according to one of Claims 17 to 21, **characterized in that** it is chosen in particular from rapeseed, crucifers, maize, soybean, wheat, sunflower, pea, ornamental plants, and trees.

23. Seeds obtained from a transgenic plant according to one of Claims 17 to 22, **characterized in that** they do not contain the product of expression of the transgene, the said seeds comprising a cassette according to one of Claims 6 to 10 or a vector according to Claim 11.


### LISTAGE DE SEQUENCE

<110> INSTITUT NATIONAL DE RECHERCHE AGRONOMIQUE - INRA
<120> PROMOTEUR PERMETTANT L'EXPRESSION DE TRANSGENES DANS TOUTE LA PLANTE HORMIS DANS LA GRAINE
<130> FAH
<140>
<141>
<160> 5
<170> PatentIn Vers. 2.0
<210> 1
<211> 932
<212> ADN
<213> Arabidopsis thaliana
<220>
<223> promoteur de la FAH chez Arabidopsis thaliana.
<400> 1
   cagctgtagc atcttgatat tgctgatact cagccacaag atcgttcatg ttactctctg 60
   cttcattaaa ctccatctcg tccattcctt cttctgtgta ccaatgcaag aaagcttatc 120
   tcaacatcag gctgatataa ccaatatctt acttctttta catttgtgaa atggaaccaa 180
   cccatttttc tggaaaaagt gctaaccaaa catttgatta accgtatcac tactactttc 240
   atttctatct tctgtttcat tatgctgact atttaagctc cgttgtcaaa tctctaagtt 300
   agacataaaa gacaaagact aatcaattgt catcacacca gcgtcgtcga gtgagctata 360
   ttaatcgtgg attttaagca ttaaagaaac attctatagt actaaagcaa ataaaataat 420
   tataatcaaa cactatgctt gacactggtc acgtgtactg gtagtgaatg attctacatc 480
   ataagaggcc gcatcaaaat cctaaaaata agcataatga attaatcatt tacaaatttt 540
   attttactca ataagaaaat cgaaagtatg attattatct agctgccaca atcttcgaat 600
   ttaatattta ctcaagaaga gaccgacttt aatccttgac tttctcattg ctctatggaa 660
   aatgattaaa gcagtcaata aaatcttttg acattgttgg cagaagacca ataattcgaa 720
   gtctaaaatg taatcgtcca cacagtgtat gagtatccta gtattttttt tcttttccat 780
   ataagttgaa tttgtaatat atatagtgta atgttgttta tttgtggcaa cgtacaaaat 840
   tgggaatcct ataagtgcga cgacaagtga caagacgagg ctatgaacag ctaatgtatg 900
   aagagagcca aaagagcaac aacctggcac ag 932
<210> 2
<211> 20
<212> ADN
<213> Artificial Sequence
<220>
<223> Amorce upper
<400> 2
   ttcatgttac tctctgcttc 20
<210> 3
<211> 23
<212> ADN
<213> Arabidopsis thaliana
<220>
<223> Amorce lower
<400> 3
   ggaaaggaaa caaatacgga ttc 23
<210> 4
<211> 237
<212> PRT
<213> Arabidopsis thaliana
<220>
<223> fatty acid hydroxylase Fah 1p
<400> 4
   Met Val Ala Gln Gly Phe Thr Val Asp Leu Lys Lys Pro Leu Val Phe
   1               5                   10                  15
   Gln Val Gly His Leu Gly Glu Asp Tyr Glu Glu Trp Val His Gln Pro
               20                  25                  30
   Ile Ala Thr Lys Glu Gly Pro Arg Phe Phe Gln Ser Asp Phe Trp Glu
           35                  40                  45
   Phe Leu Thr Leu Thr Val Trp Trp Ala Val Pro Val Ile Trp Leu Pro
       50                  55                  60
   Val Val Val Trp Cys Ile Ser Arg Ser Val Ser Met Gly Cys Ser Leu
   65                  70                  75                  80
   Pro Glu Ile Val Pro Ile Val Val Met Gly Ile Phe Ile Trp Thr Phe
                   85                  90                  95
   Phe Glu Tyr Val Leu His Arg Phe Val Phe His Ile Lys Thr Lys Ser
               100                 105                 110
   Tyr Trp Gly Asn Thr Ala His Tyr Leu Ile His Gly Cys His His Lys
           115                 120                 125
   His Pro Met Asp His Leu Arg Leu Val Phe Pro Pro Thr Ala Thr Ala
       130                 135                 140
   Ile Leu Cys Phe Pro Phe Trp Asn Ile Ala Lys Ala Ile Ser Thr Pro
   145                 150                 155                 160
   Ser Thr Ala Pro Ala Leu Phe Gly Gly Gly Met Leu Gly Tyr Val Met
                   165                 170                 175
   Tyr Asp Val Thr His Tyr Tyr Leu His His Ala Gln Pro Thr Arg Pro
               180                 185                 190
   Val Thr Lys Asn Leu Lys Lys Tyr His Leu Asn His His Phe Arg Ile
           195                 200                 205
   Gln Asp Lys Gly Phe Gly Ile Thr Ser Ser Leu Trp Asp Ile Val Phe
       210                 215                 220
   Gly Thr Leu Pro Thr Thr Lys Ala Pro Arg Lys Glu Gln
   225                 230                 235
<210> 5
<211> 714
<212> ADN
<213> Arabidopsis thaliana
<220>
<223> Sequence codante pour la fatty acid hydroxylase Fah 1P
<400> 5
   atggttgctc agggattcac tgtggatctt aaaaagcccc ttgtatttca ggttggtcat 60
   cttggagaag attatgagga atgggttcac caacctatcg cgaccaagga aggccctcgg 120
   ttttttcaga gtgacttttg ggagttcttg acacttacag tttggtgggc agttcctgtc 180
   atttggttgc cagttgtagt ctggtgcata tcaaggtcag taagtatggg atgttcactt 240
   ccagaaatcg tcccaattgt tgtcatggga atattcatct ggacattttt tgaatacgtt 300
   cttcaccggt tcgttttcca cataaaaacg aagagttact ggggaaacac tgcacactat 360
   cttattcacg gatgccatca taagcacccg atggaccacc ttcggctcgt ctttcctcct 420
   actgcaactg cgattttatg ctttccgttc tggaacattg cgaaggctat ctcaactcct 480
   tcaaccgcac ctgcattgtt tggtggaggc atgctcggat atgtgatgta cgatgtcact 540
   cattattacc ttcaccatgc ccaacctact agaccagtga ccaaaaatct caagaagtac 600
   catttgaatc atcacttcag gattcaggac aaaggatttg gtataacttc gtcgttatgg 660
   gacatagtct ttgggacact tcccaccaca aaagccccca gaaaagagca atag       714